# EUROPÄISCHE PATENTSCHRIFT

(11) **EP 2 366 330 B1**
(45) Veröffentlichungstag und Bekanntmachung des Hinweises auf die Patenterteilung: **26.11.2014**
(21) Anmeldenummer: 11156076.9
(22) Anmeldetag: 25.02.2011
(51) Int. Cl.: A61B 5/107, A43B 7/14, A43B 17/00, A43D 1/02

(54) **Verfahren und Anordnung von sensomotorischen Einlagen mit Stimulationselementen**
Method and assembly of sensorimotor inlays with stimulation elements
Procédé et agencement de semelles sensori-motrices dotées d'éléments de stimulation

(30) Priorität: 16.03.2010 DE 102010002913
(43) Veröffentlichungstag der Anmeldung: 21.09.2011
(73) Patentinhaber: medi GmbH & Co. KG, 95448 Bayreuth (DE)
(72) Erfinder: Kühnreich, Heinz-Peter, 53844 Troisdorf (DE)
(74) Vertreter: Lindner Blaumeier Patent- und Rechtsanwälte

(56) Entgegenhaltungen:
- DE-U1- 20 320 623
- US-A1- 2009 071 038
- BREUER-KÜHNREICH, ANNETTE: "Variables Einlagen Konzept für Haltung und Bewegung", ORTHOPÄDIESCHUHTECHNIK, Nr. 4, 2008, Seiten 22-24, XP002641872,
- GRUBER J.: "Anwendungsbeobachtung der IGLI interaktiv Carboneinlage im Leistungssport", ORTHOPÄDIE-TECHNIK, Bd. 2, 2008, Seiten 96-97, XP002641873,

## Beschreibung

Die Erfindung betrifft ein Verfahren und eine Anordnung zur Anpassung von sensomotorischen Einlagen mit Stimulationselementen.

Für die Motorik, insbesondere für die Erhaltung bzw. die Wiedergewinnung des Gleichgewichts spielt die sensorische Leistungsfähigkeit der plantaren Fußfläche eine wesentliche Rolle. Die Empfindlichkeit der Fußsohle lässt mit zunehmendem Alter nach. Auch bei Krankheitsbildern wie Morbus Parkinson ist eine Einschränkung der sensorischen Fähigkeiten feststellbar. Durch Stimulation der Fußsohle soll eine Verbesserung der sensomotorischen Leistungsfähigkeit erreicht werden. Dazu sind sensomotorische Einlagen oder Therapiesohlen für Schuhe bekannt, welche Überhöhungen aufweisen, welche keine Stützfunktion erfüllen, sondern rein zur Stimulation der Fußsohlen dienen. Die Anfertigung der Einlagen erfordert in jedem Fall eine individuelle Anpassung. Häufig kommt im Rahmen einer Therapie eine Abfolge einer Mehrzahl unterschiedlicher Positionierungen der Stimulationselemente zum Einsatz, was insbesondere auch bei jedem Wechsel der Positionierung eine erneute Anpassung der sensomotorischen Einlage erfordert, um mögliche Auswirkungen der vorangegangenen Therapie berücksichtigen zu können. Es ist wünschenswert, die Stimulation häufig zu verändern, so dass ein Bedarf besteht, neue Einlagen schnell und ohne unnötigen Aufwand anfertigen zu können.

Eine individuelle Anpassung erfolgt, indem ein Therapeut die Positionierung der Stimulationselemente auf Grund seiner Kenntnisse festlegt, wozu in der Regel eine Vermessung und/oder Abbildung der Fußsohlen der jeweiligen Person notwendig ist, sowie eine Ermittlung der Auswirkungen verschiedener Positionierungen auf diese Person. Diejenige Positionierung der Stimulationselemente, welche die gewünschte Auswirkung erzielt wird dann als Einlage für die Person in Auftrag gegeben und nach den Vorgaben durch einen entsprechenden Fachbetrieb hergestellt. Das Verfahren zur Ermittlung der Positionierung ist aufwändig und zeitintensiv. Darüber hinaus birgt die Schnittstelle zwischen Therapeut und Hersteller der Einlage eine gewisse Gefahr von Fehlern bei der Übermittlung der Positionierung und verlängert die Zeit bis zur Auslieferung der fertigen Einlage.

DE 203 20 623 A1 offenbart eine Vorrichtung zur Erstellung von Therapiesohlen, bestehend aus einer Plattform mit einem Scanner zum Scannen der Fußunterseiten eines Probanden. Der Vorrichtung ist eine Daten übertragende Verbindung zur Übermittlung des gescannten Bildes an einen Rechner und ein Datenverarbeitungsprogramm des Rechners, das die übermittelten Daten zu Arbeitsbefehlen einer Steuerung für eine Fräsmaschine verarbeitet, zugehörig. Auf dem Scanner sind unter dem nach einer vorgegebenen Orientierung ausgerichteten Fuß des Probanden neurologische Prozeptoren entsprechend einer Prädisposition des Probanden angeordnet.

US 2009/0071038 A1 offenbart eine Vorrichtung zur Erfassung bzw. Vermessung der Fußunterseite respektive der Fußsohle einer Person. Die Vorrichtung umfasst einen Grundkörper, von welchem eine eine Sensormatte aufweisende Verlängerung abragt. Die Sensormatte umfasst Drucksensoren und ist dazu vorgesehen, ein Abbild des Fußes einer Person zu erzeugen.

Eine Aufgabe der Erfindung besteht darin, die individuelle Anpassung der Positionierung der Stimulationselemente durch den Therapeuten mittels eines Verfahrens und einer Anordnung zur Anpassung von sensomotorischen Einlagen mit Stimulationselementen zu unterstützen, so dass diese effizienter, gegebenenfalls weniger fehleranfällig und schneller erfolgen kann.

Die Aufgabe wird durch das Verfahren und die Anordnung gemäß der unabhängigen Patentansprüche gelöst. In den Unteransprüchen sind bevorzugte Ausführungsformen und vorteilhafte Weiterbildungen angegeben.

Das Verfahren zur Anpassung von sensomotorischen Einlagen mit Stimulationselementen weist erfindungsgemäß folgende Verfahrensschritte auf:
a. Erstellen eines Abbilds der Fußsohlen einer stehenden Person, für die die sensomotorischen Einlagen gefertigt werden sollen;
b. Darstellen des Abbilds in Originalgröße auf einer Anzeige; Während der Darstellung des Abbilds auf der Anzeige erfolgen die folgenden Verfahrensschritte c1 und c2;
c1. Anordnen von Stimulationselementen auf der Anzeige gemäß einer vorgesehenen Positionierung relativ zu den in dem Abbild dargestellten Fußsohlen;
c2. Anwenden der Stimulationselemente durch die auf der Anzeige stehende Person, wobei die Füße der Person entsprechend den in dem Abbild dargestellten Fußsohlen ausgerichtet werden;
d. Verwenden der Positionierung der Stimulationselemente relativ zu dem auf der Anzeige (2) dargestellten Abbild (3') zur Herstellung sensomotorischer Einlagen mit entsprechend positionierten Stimulationselementen.

Das Erstellen des Abbilds in Schritt a erfolgt mittels einer geeigneten Bildaufnahmevorrichtung, wie insbesondere einem Scanner oder durch ein fotografisches Verfahren. Bevorzugt wird ein digitales Abbild erstellt oder das erstellte Abbild digitalisiert. Weiterhin bevorzugt wird ein zweidimensionales Abbild erstellt, was wesentlich kostengünstiger ist, als dreidimensionale Abbilder zu erstellen. Grundsätzlich ist jedoch im Rahmen der Erfindung auch das Erstellen eines dreidimensionalen Abbilds denkbar.

Gemäß Schritt b gibt das dargestellte Abbild gibt die Fußsohlen in einem originalgetreuen Maßstab (1:1) wieder, vorzugsweise auf einem geeigneten Monitor.

Die in Schritt c1 zu verwendenden Stimulationselemente können im einfachsten Fall die gleichen sein, die bei der Herstellung von sensomotorischen Einlagen benutzt werden. Vorzugsweise können die Stimulationselemente speziell für die Anpassung ausgeführt sein, beispielsweise mit einer rutschhemmenden Beschichtung und/oder mit einem Drucksensor, was später noch näher erläutert wird. Es handelt sich bei den Stimulationselementen um dreidimensionale Körper in unterschiedlichen Formen und Größen, in der Regel aus einem Kunststoff. Diese werden hier zu Anpassungszwecken verwendet. Bei der Herstellung der sensomotorischen Einlage werden später in Form und Größe gleichartige Stimulationselemente verwendet, nicht die für die Anpassung speziell ausgerüsteten Stimulationselemente, obwohl dies prinzipiell möglich wäre. Die vorgesehene Positionierung der Stimulationselemente ist durch den Therapeuten nach dessen Expertenwissen vorgegeben und nicht Teil des erfindungsgemäßen Verfahrens. Dadurch, dass ein Abbild der Fußsohlen in Originalgröße auf der Anzeige sichtbar ist, können die Stimulationselemente ohne großen Aufwand und mit großer Genauigkeit relativ zu dem Abbild auf der Anzeige positioniert werden. Auf der Anzeige liegen dann die Stimulationselemente so angeordnet, wie sie gegebenenfalls später in der sensomotorischen Einlage positioniert sind. Die Person kann besonders vorteilhaft leicht seine Position finden und einnehmen, da nach wie vor die Fußsohlen auf der Anzeige sichtbar sind, auf welche die Person sich deckungsgleich stellt. Die Anzeige ist dazu im Wesentlichen waagerecht ausgerichtet und verfügt über eine bruchsichere, durch Personen begehbare und durchsichtige Oberfläche. Hierzu kann beispielsweise in vorteilhaft einfacher Ausführung ein Flachbildschirm unmittelbar unterhalb einer Sicherheitsglasplatte angeordnet sein.

Die auf der Anzeige angeordneten Stimulationselemente können so unmittelbar an der zu therapierenden Person getestet werden. Die Anwendung der Stimulationselemente in Schritt c2 besteht im Wesentlichen darin, dass die Person sich so auf die Anzeige stellt, dass seine Füße möglichst deckungsgleich zu dem Abbild seiner Fußsohlen ausgerichtet sind, wodurch die Stimulationselemente in vorteilhafter Weise gerade diejenigen Regionen der Fußsohlen stimulieren, welche nach der vorgegebenen Positionierung vorgesehen sind. Die Anwendung erfolgt daher ausdrücklich nicht in therapeutischer Art, sondern zur Vorbereitung einer nachfolgenden, durch medizinisch ausgebildetes Fachpersonal vorzunehmenden Einschätzung der Auswirkung der Stimulationselemente auf die Person, welche nicht Bestandteil des erfindungsgemäßen Verfahrens ist. Das Ausrichten der Stimulationselemente in verschiedenen Positionierungen kann so vorteilhaft jeweils anhand des Abbilds der Fußsohlen auf der Anzeige erfolgen, so dass jeweils anschließend die nicht zum Verfahren zählende Bewertung der jeweiligen Positionierung vorteilhaft einfach und schnell durchgeführt werden kann, indem die Person sich wiederholt so auf die Anzeige stellt, dass das Abbild überdeckt wird. Die Verfahrensschritte c1 und c2 können also beliebig oft wiederholt werden. In der Regel wird durch das medizinische Fachpersonal nach deren Expertenwissen eine der Positionierungen der Stimulationselemente ausgewählt, welche in der sensomotorische Einlage zum Einsatz kommen soll. Diese Auswahl ist nicht Teil des erfindungsgemäßen Verfahrens.

In Schritt d wird die ausgewählte Positionierung der Stimulationselemente zur Herstellung der sensomotorischen Einlagen verwendet, beispielsweise als Vorlage für einen Hersteller orthopädischer Einlagen. Es ist denkbar, die Positionierung auf der Anzeige mit dem Abbild der Fußsohlen erneut abzubilden, beispielsweise zu fotografieren, um die neue Abbildung als Vorlage verwenden zu können. Dazu wird vorzugsweise ein Maßstab mit abgebildet oder erneut ein Abbild im Originalmaßstab erzeugt.

Das erfindungsgemäße Verfahren umfasst keine therapeutischen und/oder diagnostischen Maßnahmen, sondern dient allenfalls der technischen Unterstützung derselben.

Erfindungsgemäß wird in Verfahrensschritt b das Abbild mit Hilfe elektronischer Datenverarbeitung dargestellt und so bearbeitet, dass das auf der Anzeige dargestellte Abbild zusätzliche Informationen enthält, welche die vorgesehenen Positionierung der Stimulationselemente relativ zu den Fußsohlen betreffen. Ein Vorteil der elektronischen Datenverarbeitung des Abbilds besteht darin, dass diesem zusätzliche Informationen beigefügt werden können, wie beispielsweise eine Auflistung der vorgesehenen Stimulationselemente. Das Bearbeiten des Abbilds erfolgt durch Hinzufügen und/oder Verändern zusätzlicher Bildinformation, wodurch dem Abbild grafische Symbole überlagert werden, welche die Stimulationselemente repräsentieren, so dass in dem auf der Anzeige dargestellten Abbild die vorgesehene Positionierung der Stimulationselemente relativ zu den Fußsohlen sichtbar ist. Bevorzugt werden die Stimulationselemente entsprechend den grafischen Symbolen des auf der Anzeige dargestellten Abbilds positioniert. Dies hat den Vorteil, dass das Platzieren der Stimulationselemente auf der Oberfläche der Anzeige nach den eingefügten Symbolen, also nach Anleitung erfolgen kann, so dass diese Aufgabe durch Hilfskräfte durchführbar ist. Die Vorgabe der Positionierung durch medizinisches Fachpersonal, welche nicht Bestandteil des erfindungsgemäßen Verfahrens ist, erfolgt hier bereits durch die Vorgabe, wie die Bildinformation das Abbild verändern soll.

Gemäß einer weiteren bevorzugten Ausführungsform des Verfahrens wird in Verfahrensschritt d das auf der Anzeige dargestellten Abbild zur Positionierung der Stimulationselemente bei der Herstellung der sensomotorischen Einlagen verwendet. Ein Vorteil der Ausführungsform besteht darin, dass in Verfahrensschritt d das abschließend bearbeitete Abbild unmittelbar als Vorlage für die Herstellung der sensomotorischen Einlage verwendet werden kann ohne dass ein weiteres Abbild erstellt werden müsste. Das abschließend bearbeitete Abbild kann auf einem Datenträger gespeichert oder besonders vorteilhaft per Datenfernübertragung direkt an einen Hersteller orthopädischer Einlagen gesendet werden, so dass die zeitaufwändigere Übermittlung per Post entfällt.

Weiterhin bevorzugt wird zur Bearbeitung des Abbilds in Verfahrensschritt b eine Software zur Realisierung einer grafischen Benutzeroberfläche verwendet, die das Einfügen und/oder Verändern von grafischen Symbolen durch ein drag-and-drop Verfahren erlaubt. Dazu wird das Abbild insbesondere zusätzlich auf einer geeigneten Anzeigevorrichtung der EDV-Anlage angezeigt. Der Fachmann erkennt, dass so ein bereits verändertes Abbild weitergehend bearbeitet wird, indem beispielsweise die Position der grafischen Symbole verändert wird oder einzelne grafische Symbole ausgetauscht werden, oder das in Verfahrensschritt a erstellte, ursprüngliche Abbild erneut bearbeitet werden kann, wenn die Verfahrensschritte c1 und c2 wiederholt werden.

Gemäß einer bevorzugten Weiterbildung des Verfahrens beinhaltet Verfahrensschritt c2 eine dreidimensionale Bilderfassung der auf der Anzeige stehenden Person, was die Beurteilung und insbesondere den nachträglichen Vergleich der Auswirkungen verschiedener Positionierungen auf die Person erleichtert.

Alternativ oder zusätzlich umfasst Verfahrensschritt c2 eine sensorische Erfassung einer Druckbeanspruchung der Stimulationselemente auf der Anzeige. Dazu werden die Stimulationselemente vorzugsweise mit je einem insbesondere folienartigen Drucksensor versehen, welcher mindestens auf einer der Anzeige zugewandten Oberfläche angeordnet ist. Gemäß einer bevorzugten Ausführungsform werden die Verfahrensschritte b, c1 und c2 wiederholt, wobei jeweils das auf der Anzeige dargestellte Abbild und/oder die Positionierung der Stimulationselemente auf der Anzeige gespeichert wird. Die Druckwerte werden vorzugsweise angezeigt und oder mit dem Abbild für spätere Vergleiche abgespeichert. Mittels einer Software wird weiterhin bevorzugt eine Teileliste der Stimulationselemente, welche gemäß dem Abbild jeweils vorgesehen sind, erstellt und/oder aktualisiert.

Gemäß einer weiteren Ausführungsform des Verfahrens werden mittels einer Software zusätzliche, anhand des Abbilds der Fußsohlen ermittelbare Parameter bestimmt, insbesondere ein Verhältnis einer belasteten Fläche zu einer Gesamtfläche der Fußsohlen. Dies erfolgt besonders bevorzugt automatisiert durch numerische Methoden. Weiterhin bevorzugt werden mittels einer Software vorgegebene Positionierungen der grafischen Symbole in dem Abbild zur weitergehenden Bearbeitung dargestellt, beispielsweise als Vorgabe oder Startpunkt. Die Software greift dazu insbesondere auf eine gespeicherte Zuordnungstabelle zu, wobei die Zuordnungstabelle Verknüpfungen der vorgegebenen Positionierungen mit anhand des Abbilds der Fußsohlen ermittelbaren Parametern enthält. Ein bestimmtes Verhältnis von belasteter Fußfläche zur Gesamtfläche der Fußsohle führt dann zu einer Einblendung einer sinnvollen Vorauswahl von Stimulationselementen bzw. grafischen Symbolen, die dann durch medizinisches Fachpersonal nach deren Expertenwissen so verändert werden, dass eine vorgegebene Positionierung entsteht.

Das in Verfahrensschritt d zu verwendende Abbild und/oder die zu verwendende Positionierung wird vorzugsweise in Abhängigkeit von in Schritt c2 erhaltenen Erkenntnissen ausgewählt. Die Erlangung dieser Erkenntnisse obliegt medizinischem Fachpersonal und ist ausdrücklich nicht Bestandteil des erfindungsgemäßen Verfahrens. Ein dreidimensionales Abbild des Körpers der Person in Schritt c2, ein Messen der Druckverhältnisse der Stimulationselemente und die Berechnung des Verhältnisses von belasteter Fläche zu Gesamtfläche der Fußsohle können das Expertenwissen nicht ersetzen, sondern unterstützen lediglich das Fachpersonal bei seiner gegebenenfalls diagnostischen und/oder therapeutischen Tätigkeit.

Ein weiterer Gegenstand der Erfindung ist eine Anordnung zur Anpassung von sensomotorischen Einlagen mit Stimulationselementen, insbesondere nach dem zuvor beschriebenen Verfahren. Die Anordnung weist eine Bilderfassungsvorrichtung zum Erstellen eines Abbilds der Fußsohlen einer auf der Bilderfassungsvorrichtung stehenden Person, sowie eine Anzeigevorrichtung zur Darstellung des bearbeiteten Abbilds in Originalgröße auf, wobei Stimulationselemente auf der Anzeigevorrichtung relativ zu dem dargestellten Abbild entsprechend einer vorgesehenen Positionierung positionierbar sind und wobei vorgesehen ist, dass die Person derart auf der Anzeigevorrichtung steht, dass die Füße der Person mit den in dem dargestellten Abbild sichtbaren Fußsohlen in Überdeckung sind. Dazu verfügt die Anzeigevorrichtung vorzugsweise über eine waagerecht ausrichtbare, bruchfeste Oberfläche zur Bilddarstellung.

Das zuvor beschriebene Verfahren zur Anpassung von sensomotorischen Einlagen lässt sich mit der beschriebenen Anordnung besonders vorteilhaft durchführen. Weiterhin bevorzugt ist die Bilderfassungsvorrichtung direkt benachbart zu der Anzeigevorrichtung angeordnet, so dass die Person bequem von der einen Vorrichtung auf die andere herübertreten kann.

Erfindungsgemäß ist eine elektronische Datenverarbeitungsanlage mit einer Software zum Bearbeiten des Abbilds durch Hinzufügen und/oder Verändern zusätzlicher Bildinformation vorgesehen. Diese verfügt besonders bevorzugt um eine weitere Anzeige zur Darstellung und Bearbeitung des Abbilds.

Gemäß einer weiteren bevorzugten Ausführungsform weisen die Stimulationselemente auf einer der Anzeigevorrichtung zugewandten Seite ein rutschhemmendes Material und/oder einen Drucksensor auf, welcher insbesondere folienförmig gestaltet ist.

Nachfolgend wird die Erfindung anhand von Ausführungsbeispielen mit Bezug auf die beiliegenden Zeichnungen erläutert. Die Ausführungen beziehen sich sowohl auf das erfindungsgemäße Verfahren, wie auf die erfindungsgemäße Anordnung. Die Darstellung ist lediglich beispielhaft und schränkt den allgemeinen Erfindungsgedanken nicht ein.

Es zeigen
Figuren 1A, 1B, 1C, 1D einen beispielhaften Ablauf des erfindungsgemäßen Verfahrens;
Figur 2 die in dem Verfahren gemäß Figur 1 verwendeten Stimulationselemente im Detail;
Figur 3 eine Ausführungsform der erfindungsgemäßen Anordnung in einer schematischen Darstellung.

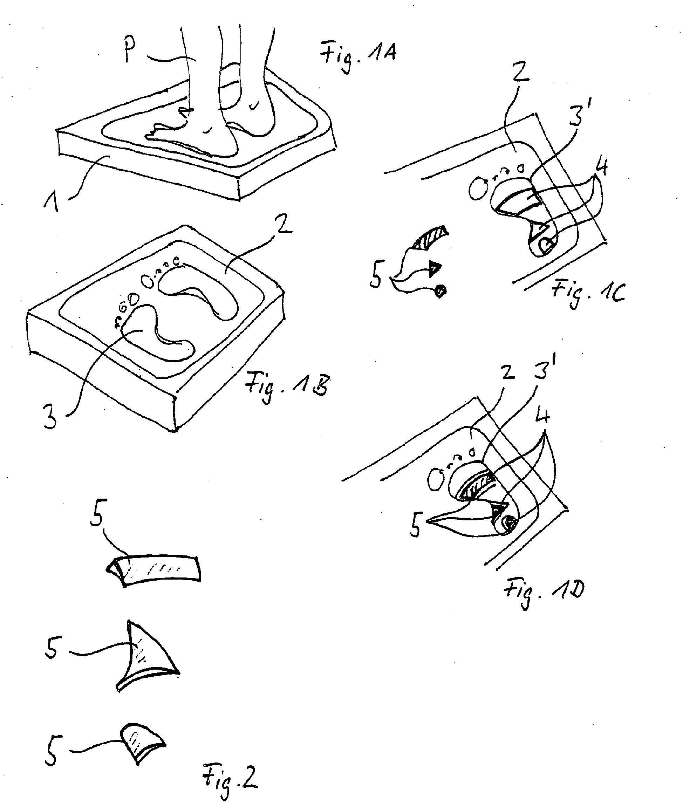

In der Figur 1A ist ein Erstellen eines Abbilds 3 (Figur 1B) der Fußsohlen einer stehenden Person P mittels einer Bilderfassungsvorrichtung 1 schematisch dargestellt. Die Bilderfassungsvorrichtung 1 ist vorzugsweise ein zweidimensionaler Scanner, welcher ein digitalisiertes Abbild 3 erzeugt. In den Figuren 1B, 1C und 1D ist jeweils eine Anzeige 2 zur Darstellung des Abbilds 3 in schematischer Darstellung gezeigt, wobei die Anzeige 2 in Figuren 1C und 1D nur ausschnittweise sichtbar ist. Das Abbild 3 wird auf der Anzeige 2 in Originalgröße, also mit Maßstab 1:1 dargestellt. In der Figur 1C ist das Abbild 3 durch grafische Symbole 4 ergänzt worden und trägt als bearbeitetes Abbild das Bezugszeichen 3' zur Unterscheidung von dem unbearbeiteten Abbild 3. Die grafischen Symbole 4 repräsentieren Stimulationselemente 5 und machen deren Größe und Positionierung relativ zu der in dem Abbild 3' erkennbaren Fußsohle sichtbar. Die Stimulationselemente 5 verfügen vorzugsweise über mindestens eine rutschhemmend ausgeführte Seite, welche der Anzeige 2 zugewandt sein soll. Die abgebildeten drei Stimulationselemente 5 sind passend zu den grafischen Symbolen 4 aus einer Auswahl weiterer Stimulationselemente verschiedener Formen und Größen herausgesucht und werden deckungsgleich mit den grafischen Symbolen 4 auf der Anzeige 2 positioniert, was in der Figur 1D dargestellt ist. Die Positionierung der grafischen Symbole kann nur nach medizinischem Expertenwissen erfolgen und ist nicht Teil der beanspruchten Gegenstände. Das Platzieren der Stimulationselemente 5 anhand der grafischen Symbole 5 kann hingegen vorteilhaft durch eine Hilfskraft ohne besondere Kenntnisse erfolgen.

Eine alternative Möglichkeit der Durchführung des Verfahrens besteht darin, dass die Stimulationselemente 5 unmittelbar auf dem nicht bearbeiteten Abbild 3 gemäß Figur 1B angeordnet werden, wobei die genaue Positionierung medizinisches Expertenwissen darstellt, was die Vornahme dieses Schritts durch Hilfspersonal ausschließt.

Die Anzeige 2 ist waagerecht ausrichtbar und insbesondere begehbar, so dass die Person P auf der Anzeige 2 stehen kann, wobei die Füße anhand des Abbilds 3, 3' ausgerichtet werden. Die dann auf der Anzeige 2 mit den Stimulationselementen 5 stehende Person P kann dann diagnostisch beurteilt werden, was nicht Gegenstand der Erfindung ist.

In der Figur 2 sind die Stimulationselemente 5 im Detail gezeigt. Diese sind vergleichbar mit solchen Stimulationselementen, welche in sensomotorischen Einlagen zum Einsatz kommen, werden hier jedoch nur für die Anpassung zu Testzwecken verwendet. Dazu sind die Stimulationselemente 5 vorzugsweise speziell ausgestattet, insbesondere mit einer rutschhemmenden Beschichtung auf mindestens einer Seite um ein Haften an der Oberfläche der Anzeige 2 (Figur 1D) zu gewährleisten. Zusätzlich oder alternativ weisen die Stimulationselemente 5 einen Drucksensor in Form einer sehr dünnen Sensorfolie auf, welche auf mindestens einer Oberfläche angeordnet ist.

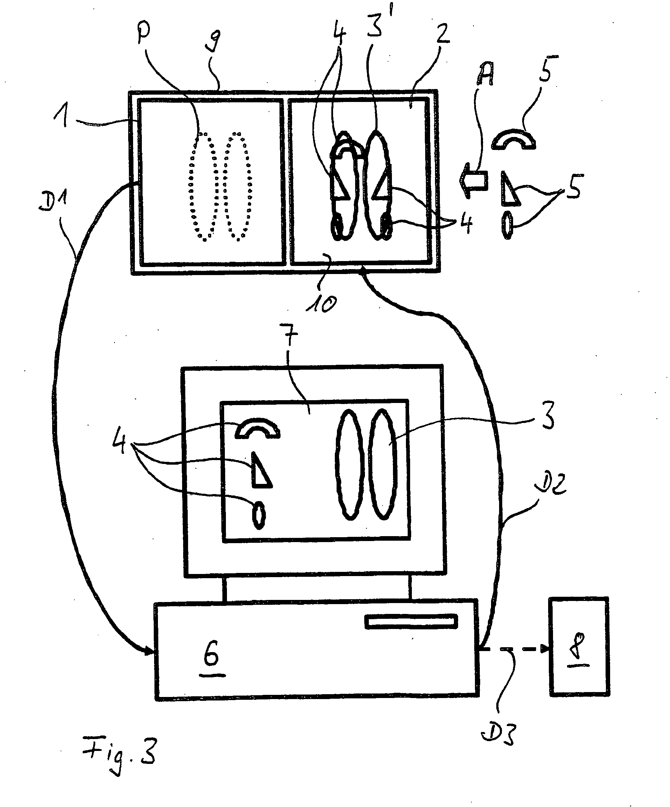

In der Figur 3 ist eine schematische Übersicht einer Ausführungsform einer erfindungsgemäßen Anordnung dargestellt. Kernstück der Erfindung stellen die Bilderfassungsvorrichtung 1 und die Anzeige 2 auf, welche beide begehbar ausgeführt sind, wobei die Anzeige 2 beispielsweise durch einen Monitor unterhalb einer Glasplatte 10, insbesondere aus Sicherheitsglas, realisierbar ist. Begehbare Fußscanner als Bilderfassungsvorrichtung 1 sind im Bereich der Orthopädie bereits erhältlich. Für die Person P, deren Fußsohle auf dem Scanner 1 in unterbrochener Linie dargestellt ist, ist es besonders angenehm und bequem, wenn die waagerecht ausrichtbare Anzeige 2 unmittelbar neben dem Scanner 1 aufgestellt ist, bzw. diese in einem gemeinsamen Gehäuse 9 angeordnet sind und die Person mit einem kurzen Schritt von einem Gerät auf das andere steigen kann.

Das eingescannte digitalisierte Abbild 3 wird von dem Scanner1 über eine erste Datenleitung D1 an eine Anlage zur elektronischen Datenverarbeitung 6 übertragen. Bei der EDV-Anlage 6 handelt es sich in der Regel um einen für eine Büroausstattung üblichen Computer mit einem Monitor 7, auf welchem das Abbild 3 der Fußsohlen anzeigbar ist. Die Anzeige kann auf dem Monitor 7 in Originalgröße erfolgen, was jedoch nicht zwingend erforderlich ist. Mit Hilfe einer Software, wird dem Abbild 3 Bildinformation in Form von grafischen Symbolen 4, welche das Abbild 3 überlagern, hinzugefügt. Dies erfolgt vorzugsweise mittels einer graphischen Benutzeroberfläche, welche dem Bediener das Verändern des Abbilds und der grafischen Symbole durch ein sogenanntes Drag-and-Drop Verfahren erlaubt.

Das derart veränderte Abbild 3' wird über eine zweite Datenleitung D2 an die Anzeige 2 übertragen. Die grafischen Symbole 4 werden mit passenden Stimulationselementen 5 belegt, was durch den Pfeil A angedeutet ist. Die nicht dargestellte Person kann nun so auf der Anzeige 2 stehen, dass ihre Füße die in dem Abbild 3' sichtbaren Fußsohlen überdecken, wodurch die Person automatisch auf den nach vorgegebener Positionierung angeordneten Stimulationselementen 5 steht und die Wirkung derselben beurteilbar ist.

Das Verfahren kann mehrfach wiederholt werden, wobei die Person jeweils beiseite tritt, so dass die Stimulationselemente 5 neu gemäß veränderten grafischen Symbolen 4 positioniert werden können. Die neue Positionierung entspricht dann einer veränderten Vorgabe, welche am Computer 6 eingegeben wurde. Die verschiedenen Positionierungen können anhand der jeweils zugehörigen Abbilder 3' auf einem Speichermedium des Computers 6 gespeichert werden, vorzugsweise mit zusätzlichen Informationen zur Bewertung der jeweiligen Positionierung. Schließlich wird in der Regel eine Positionierung, gegebenenfalls auch mehrere Positionierungen für die Herstellung einer sensomotorischen Einlage ausgewählt. Besonders vorteilhaft kann das zugehörige Abbild 3' als Vorlage für die Herstellung an den Hersteller übermittelt werden, vorzugsweise verzugslos durch Datenfernübertragung über eine Verbindung D3 auf einen Server 8 des Herstellers. Dieser kann somit unmittelbar nach Erhalt des Abbilds 3' als Vorlage mit der Herstellung einer so angepassten Einlage beginnen.

## Patentansprüche

1. Verfahren zur Anpassung von sensomotorischen Einlagen mit Stimulationselementen, mit den folgenden Verfahrensschritten:
a. Erstellen eines Abbilds (3) der Fußsohlen einer stehenden Person (P);
b. Darstellen des Abbilds (3') in Originalgröße auf einer Anzeige (2) und während der Darstellung des Abbilds:
c1. Anordnen von Stimulationselementen (5) auf der Anzeige (2) gemäß einer vorgesehenen Positionierung relativ zu den in dem Abbild (3') dargestellten Fußsohlen;
c2. Anwenden der Stimulationselemente (5) durch die auf der Anzeige (2) stehende Person (P), wobei die Füße der Person entsprechend den in dem Abbild (3') dargestellten Fußsohlen ausgerichtet werden;
d. Verwenden der Positionierung der Stimulationselemente relativ zu dem auf der Anzeige (2) dargestellten Abbild (3') zur Herstellung sensomotorischer Einlagen mit entsprechend positionierten Stimulationselementen,
wobei
in Verfahrensschritt b das Abbild (3) mit Hilfe elektronischer Datenverarbeitung (6) dargestellt und so bearbeitet wird, dass das auf der Anzeige dargestellte Abbild (3') zusätzliche Informationen zu der vorgesehenen Positionierung der Stimulationselemente (5) relativ zu den Fußsohlen enthält,
wobei
das Bearbeiten des Abbilds (3) durch Hinzufügen und/oder Verändern zusätzlicher Bildinformation erfolgt, wodurch dem Abbild (3) grafische Symbole (4) überlagert werden, welche Stimulationselemente (5) repräsentieren, so dass in dem auf der Anzeige (2) dargestellten Abbild (3') die vorgesehene Positionierung der Stimulationselemente (5) relativ zu den Fußsohlen sichtbar ist.

2. Verfahren nach Anspruch 1,
**dadurch gekennzeichnet,**
**dass** die Stimulationselemente (5) entsprechend den grafischen Symbolen (4) des auf der Anzeige (2) dargestellten Abbilds (3') positioniert werden;

3. Verfahren nach einem der vorhergehenden Ansprüche,
**dadurch gekennzeichnet,**
**dass** in Verfahrensschritt d das auf der Anzeige (2) dargestellte Abbild (3') zur Positionierung der Stimulationselemente (5) bei der Herstellung der sensomotorischen Einlagen verwendet wird.

4. Verfahren nach einem der vorhergehenden Ansprüche,
**dadurch gekennzeichnet,**
**dass** Verfahrensschritt c2 eine dreidimensionale Bilderfassung der auf der Anzeige (2) stehenden Person (P) beinhaltet.

5. Verfahren nach einem der vorhergehenden Ansprüche,
**dadurch gekennzeichnet,**
**dass** Verfahrensschritt c2 eine sensorische Erfassung einer Druckbeanspruchung der Stimulationselemente (5) auf der Anzeige (2) beinhaltet.

6. Verfahren nach einem der vorhergehenden Ansprüche,
**dadurch gekennzeichnet,**
**dass** die Verfahrensschritte b, c1 und c2 wiederholt werden, wobei jeweils das auf der Anzeige (2) dargestellte Abbild (3') und/oder die Positionierung der Stimulationselemente (5) auf der Anzeige (2) gespeichert wird.

7. Verfahren nach einem der vorhergehenden Ansprüche,
**dadurch gekennzeichnet,**
**dass** in Verfahrensschritt d das zu verwendende Abbild (3') und/oder die zu verwendende Positionierung in Abhängigkeit von in Schritt c2 erhaltenen Erkenntnissen ausgewählt wird.

8. Verfahren nach einem der vorhergehenden Ansprüche,
**dadurch gekennzeichnet,**
**dass** mittels einer Software zusätzliche, anhand des Abbilds (3) der Fußsohlen ermittelbare Parameter bestimmt werden, insbesondere ein Verhältnis einer belasteten Fläche zu einer Gesamtfläche der Fußsohlen.

9. Verfahren nach einem der vorangehenden Ansprüche,
**dadurch gekennzeichnet,**
**dass** mittels einer Software durch Zugreifen auf eine gespeicherte Zuordnungstabelle vorgegebene Positionierungen der grafischen Symbole (4) in dem Abbild (3) zur weitergehenden Bearbeitung dargestellt werden, wobei die Zuordnungstabelle Verknüpfungen der vorgegebenen Positionierungen mit anhand des Abbilds (3) der Fußsohlen ermittelbaren Parametern enthält.

10. Verfahren nach einem der vorhergehenden Ansprüche,
**dadurch gekennzeichnet,**
**dass** mittels einer Software eine Teileliste der Stimulationselemente (5), welche gemäß Abbild (3') vorgesehen sind, erstellt und/oder aktualisiert wird.

11. Anordnung zur Anpassung von sensomotorischen Einlagen mit Stimulationselementen, aufweisend eine Bilderfassungsvorrichtung (1) zum Erstellen eines Abbilds (3) der Fußsohlen einer auf der Bilderfassungsvorrichtung stehenden Person (P),
eine Anzeigevorrichtung (2) zur Darstellung des bearbeiteten Abbilds (3') in Originalgröße,
wobei Stimulationselemente (5) auf der Anzeigevorrichtung (2) relativ zu dem dargestellten Abbild (3') entsprechend einer vorgesehenen Positionierung positionierbar sind und wobei vorgesehen ist, dass die Person (P) derart auf der Anzeigevorrichtung (2) steht, dass die Füße der Person mit den in dem dargestellten Abbild (3') sichtbaren Fußsohlen in Überdeckung sind,
**dadurch gekennzeichnet,**
**dass** eine elektronische Datenverarbeitungsanlage (6) mit einer Software zum Bearbeiten des Abbilds (3) durch Hinzufügen und/oder Verändern zusätzlicher Bildinformation vorgesehen ist, wobei die Software zum Bearbeiten des Abbilds (3) durch Hinzufügen und/oder Verändern zusätzlicher Bildinformation derart ausgebildet ist, dass dem Abbild (3) grafische Symbole (4) überlagert werden, welche Stimulationselemente (5) repräsentieren, so dass in dem auf der Anzeige (2) dargestellten Abbild (3') die vorgesehene Positionierung der Stimulationselemente (5) relativ zu den Fußsohlen sichtbar sind.

12. Anordnung nach Anspruch 11,
**dadurch gekennzeichnet,**
**dass** die Stimulationselemente (5) auf einer der Anzeigevorrichtung (2) zugewandten Seite ein rutschhemmendes Material aufweisen und/oder einen Drucksensor.

## Claims

1. Method for adapting sensorimotor inlays with stimulation elements, comprising the following method steps:
a. creating an image (3) of the soles of the feet of a standing person (P);
b. representing the image (3') in the original size on a display (2) and, during the representation of the image:
c1. arranging stimulation elements (5) on the display (2) in accordance with a provided positioning relative to the soles of the feet represented in the image (3');
c2. applying the stimulation elements (5) by the person (P) standing on the display (2) wherein the person's feet are aligned in a manner corresponding to the soles of the feet represented in the image (3');
d. using the positioning of the stimulation elements relative to the image (3') represented on the display (2) to produce sensorimotor inlays with correspondingly positioned stimulation elements,
wherein
in method step b the image (3) is represented and edited with the aid of electronic data processing (6) such that the image (3') represented on the display contains additional information concerning the provided positioning of the stimulation elements (5) relative to the soles of the feet,
where
the image (3) is edited by additional image information being added and/or altered, as a result of which graphical symbols (4) representing stimulation elements (5) are superimposed on the image (3), such that the provided positioning of the stimulation elements (5) relative to the soles of the feet is visible in the image (3') represented on the display (2).

2. Method according to Claim 1,
**characterized**
**in that** the stimulation elements (5) are positioned in a manner corresponding to the graphical symbols (4) of the image (3') represented on the display (2).

3. Method according to either of the preceding claims,
**characterized**
**in that** in method step d the image (3') represented on the display (2) is used for positioning the stimulation elements (5) during the production of the sensorimotor inlays.

4. Method according to any of the preceding claims,
**characterized**
**in that** method step c2 includes a three-dimensional image acquisition of the person (P) standing on the display (2).

5. Method according to any of the preceding claims,
**characterized**
**in that** method step c2 includes a sensor-based acquisition of a pressure loading of the stimulation elements (5) on the display (2).

6. Method according to any of the preceding claims,
**characterized**
**in that** method steps b, c1 and c2 are repeated, the image (3') represented on the display (2) and/or the positioning of the stimulation elements (5) on the display (2) being stored in each case.

7. Method according to any of the preceding claims,
**characterized**
**in that** in method step d the image (3') to be used and/or the positioning to be used are/is selected depending on insights obtained in step c2.

8. Method according to any of the preceding claims,
**characterized**
**in that** additional parameters which can be ascertained on the basis of the image (3) of the soles of the feet, in particular a ratio of a loaded area to a total area of the soles of the feet, are determined by means of software.

9. Method according to any of the preceding claims,
**characterized**
**in that**, by means of software, by a stored assignment table being accessed, predefined positioning of the graphical symbols (4) are represented in the image (3) for more extensive editing, wherein the assignment table contains combinations of the predefined positioning with parameters that can be ascertained on the basis of the image (3) of the soles of the feet.

10. Method according to any of the preceding claims,
**characterized**
**in that** a list of parts of the stimulation elements (5) which are provided in accordance with the image (3') is created and/or updated by means of software.

11. Arrangement for adapting sensorimotor inlays with stimulation elements, comprising an image acquisition device (1) for creating an image (3) of the soles of the feet of a person (P) standing on the image acquisition device,
a display device (2) for representing the edited image (3') in the original size,
wherein stimulation elements (5) can be positioned on the display device (2) relative to the represented image (3') in a manner corresponding to a provided positioning, and wherein provision is made for the person (P) to stand on the display device (2) in such a way that the person's feet coincide with the soles of the feet visible in the represented image (3'),
**characterized**
**in that** provision is made of an electronic data processing system (6) comprising software for editing the image (3) by adding and/or altering additional image information, wherein the software for editing the image (3) by adding and/or altering additional image information is designed in such a way that graphical symbols (4) representing stimulation elements (5) are superimposed on the image (3), such that the provided positioning of the stimulation elements (5) relative to the soles of the feet is visible in the image (3') represented on the display (2).

12. Arrangement according to Claim 11,
**characterized**
**in that** the stimulation elements (5) have, on a side facing the display device (2), an anti-slip material and/or a pressure sensor.

## Revendications

1. Procédé d'adaptation d'inserts sensori-moteurs comportant des éléments de stimulation, comprenant les étapes de procédé suivantes :
a. créer une image (3) de la plante des pieds d'une personne se tenant debout (P) ;
b. représenter l'image (3') à sa taille originale sur un dispositif d'affichage (2) et pendant la représentation de l'image :
c1. agencer des éléments de stimulation (5) sur le dispositif d'affichage (2) selon un positionnement prédéterminé par rapport aux plantes des pieds représentées dans l'image (3') ;
c2. appliquer les éléments de stimulation (5) à travers la personne (P) se tenant debout sur le dispositif d'affichage (2), dans lequel les pieds de la personne sont alignés d'une manière qui correspond aux plantes des pieds représentées dans l'image (3') ;
d. utiliser le positionnement des éléments de stimulation par rapport à l'image (3') représentée sur le dispositif d'affichage (2) pour réaliser des inserts sensori-moteurs comportant des éléments de stimulation positionnés de manière correspondante, dans lequel lors de l'étape de procédé b, l'image (3) est représentée à l'aide d'un traitement de données électronique (6) et est traitée de manière à ce que l'image (3') représentée sur le dispositif d'affichage contienne des informations supplémentaires en plus du positionnement prévu des éléments de stimulation (5) par rapport aux plantes des pieds,
dans lequel
le traitement de l'image (3) est effectué par introduction et/ou modification d'informations d'images supplémentaires de manière à superposer à l'image (3) des symboles graphiques (4) qui représentent des éléments de stimulation (5) afin que le positionnement prévu des éléments de stimulation (5) par rapport aux plantes des pieds soit visible dans l'image (3') représentée sur le dispositif d'affichage (2).

2. Procédé selon la revendication 1, **caractérisé en ce que** les éléments de stimulation (5) sont positionnés d'une manière qui correspond aux symboles graphiques (4) de l'image (3') représentée sur le dispositif d'affichage (2).

3. Procédé selon l'une quelconque des revendications précédentes, **caractérisé en ce que**, lors de l'étape de procédé d, l'image (3') représentée sur le dispositif d'affichage (2) est utilisée pour positionner les éléments de stimulation (5) lors de la fabrication des inserts sensori-moteurs.

4. Procédé selon l'une quelconque des revendications précédentes, **caractérisé en ce que** l'étape de procédé (c2) comprend une acquisition d'image tridimensionnelle de la personne (P) se tenant debout sur le dispositif d'affichage (2).

5. Procédé selon l'une quelconque des revendications précédentes, **caractérisé en ce que** l'étape de procédé c2 comprend l'acquisition au moyen de capteurs d'une charge de compression des éléments de stimulation (5) sur le dispositif d'affichage (2).

6. Procédé selon l'une quelconque des revendications précédentes, **caractérisé en ce que** les étapes de procédé b, c1 et c2 sont répétées, dans lequel l'image (3') représentée sur le dispositif d'affichage (2) et/ou le positionnement des éléments de stimulation (5) sur le dispositif d'affichage (2) sont chaque fois stockés en mémoire.

7. Procédé selon l'une quelconque des revendications précédentes, **caractérisé en ce que**, lors de l'étape de procédé d, l'image (3') à utiliser et/ou le positionnement à utiliser est/sont sélectionné(s) en fonction d'informations obtenues à l'étape c2.

8. Procédé selon l'une quelconque des revendications précédentes, **caractérisé en ce que** l'on détermine au moyen d'un logiciel des paramètres supplémentaires pouvant être déterminés à partir de l'image (3) de la plante des pieds, notamment un rapport d'une surface d'appui à une surface totale de la plante des pieds.

9. Procédé selon l'une quelconque des revendications précédentes, **caractérisé en ce que**, au moyen d'un logiciel, des positionnements prédéterminés des symboles graphiques (4) dans l'image (3) sont représentés par accès à une table d'affectation stockée en mémoire en vue de la poursuite du traitement, dans lequel la table d'affectation contient des combinaisons des positionnements prédéterminés avec des paramètres pouvant être déterminés sur la base de l'image (3) de la plante des pieds.

10. Procédé selon l'une quelconque des revendications précédentes, **caractérisé en ce que**, au moyen d'un logiciel, une liste de pièces des éléments de stimulation (5) qui sont prévus conformément à l'image (3') est établie et/ou actualisée.

11. Dispositif d'adaptation d'inserts sensori-moteurs comportant des éléments de stimulation, comprenant un dispositif d'acquisition d'image (1) destiné à créer une image (3) de la plante des pieds d'une personne (P) se tenant débout sur le dispositif d'acquisition d'image,
un dispositif d'affichage (2) destiné à représenter l'image traitée (3') à sa taille originale,
dans lequel des éléments de stimulation (5) peuvent être positionnés sur le dispositif d'affichage (2) par rapport à l'image représentée (3') d'une manière qui correspond à un positionnement prévu et dans lequel il est fait en sorte que la personne (P) se tienne sur le dispositif d'affichage (2) de manière à ce que les pieds de la personne recouvrent les plantes des pieds visibles dans l'image (3') représentée,
**caractérisé en ce qu'**il est prévu un système de traitement de données électronique (6) comportant un logiciel destiné à traiter l'image (3) par introduction et/ou modification d'informations d'images supplémentaires, dans lequel le logiciel destiné à traiter l'image (3) par introduction et/ou modification d'informations d'images supplémentaires est conçu de manière à superposer à l'image (3) des symboles graphiques (4) qui représentent des éléments de stimulation (5), de manière à ce que le positionnement prévu des éléments de stimulation (5) par rapport aux plantes des pieds soit visible dans l'image (3') représentée sur le dispositif d'affichage (2).

12. Dispositif selon la revendication 11, **caractérisé en ce que** les éléments de stimulation (5) comprennent, sur une face opposée au dispositif d'affichage (2), un matériau anti-glissement et/ou un capteur de pression.
